# EUROPEAN PATENT APPLICATION

(11) **EP 1 044 955 A1**
(43) Date of publication of application: **18.10.2000**
(21) Application number: 99949385.1
(22) Date of filing: 22.10.1999
(51) Int. Cl.: C07C 67/08, C07C 67/26, C07C 69/16

(54) **PROCESSES FOR THE PREPARATION OF ORGANIC DIESTERS**

(30) Priority: 23.10.1998 JP 30310598; 23.10.1998 JP 30310698; 22.09.1999 JP 26919899
(71) Applicant: DAICEL CHEMICAL INDUSTRIES, Ltd., Sakai-shi, Osaka 590-0905 (JP)
(72) Inventor: MATUDA, Hirokazu, Himeji-shi Hyogo 671-1254 (JP); OKA, Kenji, Ohtake-shi Hiroshima 739-0605 (JP); TAKEMOTO, Etsuo, Hiroshima 739-0421 (JP); HAMANISHI, Moriaki, iwa-cho, Kuga-gun Yamaguchi 740-1231 (JP); UEMURA, Sakae, Kyoto-shi Kyoto 606-0841 (JP)
(74) Representative: Portal, Gérard
(86) International application number: JP9905863
(87) International publication number: WO0024702

(57) **Abstract**

The present invention relates a method of preparing an organic diester which comprises a first step (1) whom a diolefin and a peracid compound are allowed to react to synthesize a monoepoxide, a second step (2) wherein said synthesized monoepoxide is allowed to react with water and carboxylic acid and a third step (3) where the synthesized compounds are allowed to react with an acid anhydride. The present invention also relates to a method of preparing an organic diester wherein in the second step (2), the molar ratio of water and carboxylic acid added into monoepoxide is more than 2 and not more than 20, to an organic diester preparing method wherein monoepoxide is allowed to react with water in the second step (2) and to a method of preparing an organic diester wherein in the second step (2), water and carboxylic acid are removed from the synthesized product and recycled to be used again in said second step (2). The purpose of the present invention is to provide a method of preparing an organic diester having a good selectivity a producing few impurities and using diolefin as a starting material. In the second step (2), when water and carboxylic acid are recycled and used again, it is possible to reduce the load of dumping water and particularly, when 1, 3-butadiene is used as diolefin, acetic acid is used as carboxylic acid and acetic anhydride is used as an acid anhydride it is also possible to reduce the use amount of acetic acid.

## Description

### TECHNICAL FIELD

The present invention relates to a method of preparing an organic diester. In more detail, the present invention relates to a method of preparing an organic diester according to a process where a monoepoxide is firstly obtained from a diolefin and a peroxide, this monoepoxide obtained is then transformed into a monoester which is finally transformed into a diester. The diester obtained according to the method of the present invention contains few impurities and may be usefully used as a starting material for drugs or the like.

### TECHNICAL BACKGROUND

In synthesis of an organic diester using a diolefin as a starting material, when the reaction is a single-step reaction using a carboxylic acid or an anhydride of an acid or the like, there are a lot of by-products synthesized or the conversion ratio of the diolefin is often low.

In other words, when synthesizing an organic diester in a single step, it is possible to use a catalyst in order to improve the reactivity and the selectivity of the reaction. However, since the reaction conditions suitable for the synthesis of the monoepoxide which is an intermediate compound of the reaction, and those suitable for the synthesis of the organic monoester and the organic diester respectively are greatly different from each other, there are limits in improving the yield of the reaction.

There are two or three steps-methods for obtaining the target organic diester. These methods use a diolefin as a starting material and comprise a reaction for synthesizing an intermediate compound. However, in all of these methods, a catalyst is used and thus these methods are very complicated from an industrial point of view because of catalyst treatment or the like after reaction. Furthermore, with such methods, problems are caused regarding treatment of waste liquid and dumping water.

JP-B-75-023008 and JP-B-77-012171, Official Gazettes disclose a method for producing diacetoxybutene by reading 1, 3-butadiene with oxygen in molecular state and acetic acid using a palladium-type solid catalyst. Moreover, methods using the same reaction with rhodium-type solid catalyst are disclosed in JP-A-51-108010 and JP-A-52-139004. The selectivity of 1, 4-diacetoxy-2-butene is higher when mixing tellurium or selenium with rhodium compared to the palladium-type catalyst. However, loss of rhodium is very troublesome. Further, in order to control the loss of rhodium metal during reaction, JP-A-55-000338 Official Gazette discloses a method wherein the same reaction is carried out with a catalyst containing at least one of the elements as a necessary component selected from rhodium, antimony, arsenic and thallium. However, even in these methods, the selectivity of 3, 4-diacetoxy-1-butene into 1, 4-diacetoxy-2-butene which is a main product is low.

JP-A-56-092833 discloses another example of reaction using a catalyst. According to this method, alumino-silicates containing palladium are first synthesized and then modified with alkali metals or/and alkali earth metals to give a catalyst in which palladium content is freely adjusted. However, even according to this method, the selectivity of 3, 4-diacetoxy-1-butene into 1, 4-diacetoxy-2-butene which is a main product is low.

In addition, JP-A-54 160587 Official Gazette discloses an example in which 1,4-diacetoxy-2-butene is primarily obtained by the same reaction using a solid catalyst modified with palladium, vanadium, and iodine components onto an activated carbon. There is also an example where 3, 4-diacetoxy-1-butene is obtained more selectively than 1, 4-diacetoxy-2-butene through reacting butadiene and acetic acid with oxygen in a molecular state. According to JP-A-47 039003 and JP-A-47 039004, it is possible to obtain more selectively 3, 4-diacetoxy-1-butene than 1, 4-diacetoxy-2-butene by using an organic acid solution containing halogen ion and an organic acid solution containing halogen ion and antimony compounds respectively in the presence of a palladium catalyst. However, even when the conversion rate of 3, 4-diacetoxy-1-butene is very high, this rate is approximately twice the conversion rate of 1, 4-diacetoxy-2-butene and thus there is no reason to selectively obtain only 3, 4-diacetoxy-1-butene.

There is another method for synthesizing diacetoxybutene in one step from butadiene. This method consists of allowing to react acetic acid with oxygen in an organic acid solvent in the presence of palladium salt(s) and/or a redox-type catalysts of copper (GB-A-1 170 222). However, according to this method, palladium is uniformly dissolved in the reaction system and thus there is need for an extremely difficult step for separating, recovering and recycling palladium. Further, during this step, there are unavoidable metal losses and especially in the case of palladium which is an expensive metal, this method is not industrially suitable.

The object of the present invention is to provide a method of producing an organic diester from a diolefin which has a good selectivity, that is to say, which produces few impurities.

### DISCLOSURE OF THE INVENTION

The inventors of the present invention found as a result of an intensive investigation to solve the problems involved by methods for preparing an organic diester, that it is possible to obtain an organic diester with a higher activity and selectivity than those obtained in the prior art method. The novel method found comprises a first step where a monoepoxide is synthesized by reacting a diolefin with a peracid, a second step where the monoepoxide obtained in the first step is reacted with water or with a mixture of water and a carboxylic acid at a determined mole ratio of acid to synthesize as a major compound a diol or an organic monoester and a third step where the synthesized products are reacted with an acid anhydride. According to the present invention, it has been discovered that it is possible to obtain selectively 3, 4-diacetoxy-1-butene by using, for example, butadiene as a diolefin, peracetic acid as a peracid, acetic acid as a carboxylic acid and acetic anhydride as an acid anhydride.

The first aspect of the present invention provides a method of preparing an organic diester comprising a first step (1) where a monoepoxide is synthesized by reacting a diolefin with a peracid compound, a second step (2) where the monoepoxide is reacted with water and carboxylic acid and a third step (3) where the synthesized products are reacted with an acid anhydride. According to the present invention, in the second step (2) of the above-mentioned method, water and carboxylic acid are allowed to react with monoepoxide in molar ratio of not more than (water/carboxylic acid) 0.1 to 2.

The present invention provides a method of preparing an organic diester wherein the diolefin is butadiene.

Preferably, according to the present invention, the peracid compound is selected from at least one compound of the following compounds : hydrogen peroxide, performic acid, peracetic acid and perpropionic add.

According to the present invention, the performic acid used is preferably selected among at least one of the following acids : methanoic acid, acetic acid and propionic acid.

Further, according to the present invention, the acid anhydride used is preferably selected among at least one of the following anhydrides : formic anhydride, acetic anhydride and propionic anhydride.

According to the present invention, the diolefin used may be 1, 3-butadiene, the peracid compound may be peracetic add, the carboxylic acid may be acetic acid and the anhydride of an acid may be the acetic anhydride.

The present invention also provides a method of preparing 3, 4-diacetoxy-1-butene using 1, 3-butadiene as a diolefin, peracetic acid as a peracid, acetic acid as a carboxylic acid and acetic anhydride as an anhydride compound.

The second aspect of the present invention provides a method of preparing an organic diester preparing method which comprises a first step (1) where the monoepoxide is synthesized by reacting a diolefin with a peracid compound, a second step (2) where the monoepoxide is reacted with a mixture of water and a carboxylic acid containing a molar ratio of water/carboxylic acid of more than 2.0 and less than 20 and a third step (3) where the synthesized products are reacted with an acid anhydride. According to the second aspect of the present invention, the diolefin used in the above-mentioned method is preferably butadiene. Further, the peracid compound is at least one selected from the group consisting of hydrogen peroxide, performic acid, peracetic acid and perpropionic acid. Moreover, the acid anhydride is at least one selected from the group consisting of formic anhydride, acetic anhydride and propionic anhydride.

The second aspect of the invention also provides a method of preparing 3, 4-diacetoxy-1-butene by using 1, 3-butadiene as a diolefin, peracetic acid as a peracid compound, acetic acid as a carboxylic acid and acetic anhydride as an anhydride compound.

The third aspect of the invention provides a method of preparing an organic diester comprising a first step (1) where a monoepoxide is synthesized by reacting a diolefin with a peracid compound, a second step (2) where the monoepoxide is reacted with water and a third step (3) where the synthesized products are reacted with an acid anhydride.

According to the present invention, the diolefin used in the above-mentioned method is preferably butadiene. Further, the peracid compound is at least one compound selected from the group consisting of hydrogen peroxide, permethanoic acid, peracetic acid and perpropionic acid. Moreover, the acid anhydride is at least one selected from the group consisting of formic anhydride, acetic anhydride and propionic anhydride.

The third aspect of the invention also provides a method of preparing 3, 4-diacetoxy-1-butene through using 1, 3-butadiene as a diolefin, peracetic acid as a peracid, acetic acid as a carboxylic acid and acetic anhydride as an anhydride.

The fourth aspect of the invention provides a method of preparing an organic diester preparing method comprising a first step (1) where a monoepoxide is synthesized by reacting a diolefin with a peracid compound, a second step (2) where the monoepoxide is allowed to react with water or water and carboxylic acid, preferably with a mixture of water and carboxylic acid containing a molar ratio water/carboxylic acid of more than 2 and less than 20 and a third step (3) where the synthesized products are allowed to react with an acid anhydride, the method being characterized in that in the second step (2) water and carboxylic acid are removed from the synthesized product of the second step and recycled to be used again in the second step (2). According to the invention, the diolefin used in the above-mentioned method is preferably 1, 3-butadiene, the peracid is preferably peracetic acid, the carboxylic acid is preferably acetic acid and the anhydride is preferably acetic anhydride.

### BRIEF DESCRIPTION OF THE FIGURES

- Fig. 1 shows an example of the preparation process of the invention using 1, 3-butadiene as a starting diolefin.
- Fig. 2 relates to the second step (2) of the process and shows the preparation process of the third aspect of the invention where water is reacted. In all figures Ac refers to an acetyl group.

### BEST MODE FOR CARRYING OUT THE INVENTION

The means for carrying out of the present invention will be described hereinafter.

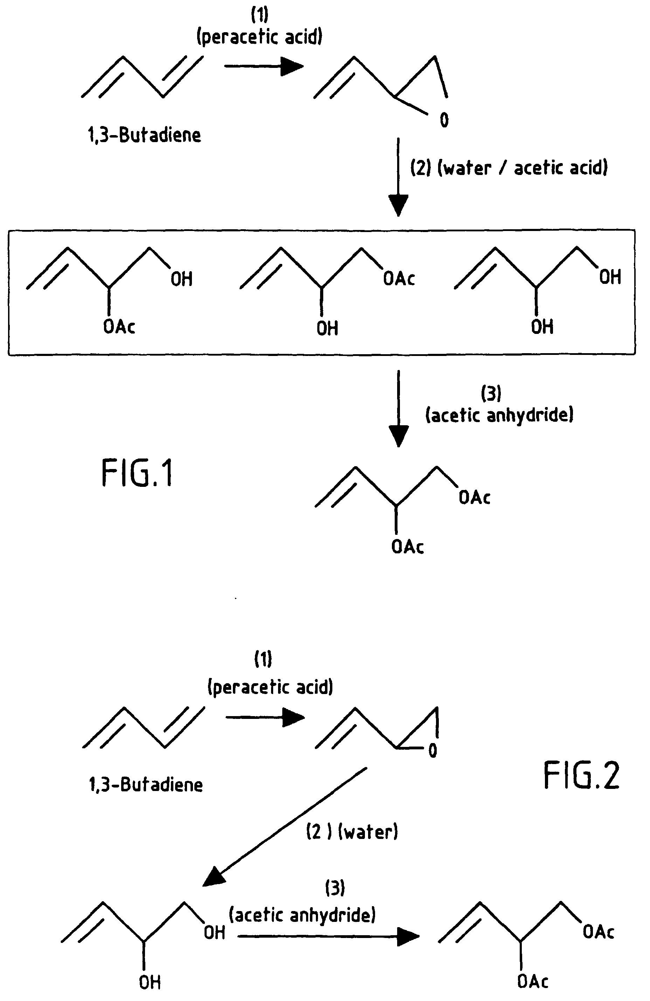

An example of the method of the invention which corresponds to the first and second aspects of the present invention with 1,3-butadiene given as an example of diolefin starting material is conform to the producing scheme shown in Fig. 1.

The first aspect of the invention comprises the step (1) of reacting 1, 3-butadiene with peracetic acid (peracid compound) to synthesize a monoepoxide ; in the second step (2) this monoepoxide synthesized is then reacted with water and acetic acid (carboxylic acid) to produce a monoester ; in a third step (3) this synthesized monoester is reacted with an anhydride of acetic acid (anhydride of an acid) to produce 3, 4-diacetoxy-1-butene.

The second aspect of the invention is a method of producing an organic diester wherein in the second step of the first aspect of the invention the synthesized monoepoxide is reacted with a mixture of water and a carboxylic acid containing a molar ratio of more than 2 and less than 20 (water / carboxylic acid).

The third aspect of the invention is a method of producing an organic diester wherein in the second step of the first aspect of the invention the monoepoxide synthesized is reacted with water.

Further, the fourth aspect of the invention is a method of producing an organic diester wherein water and carboxylic acid are recovered from the reaction liquid mixture in the second step (2) and recycled into the second step (2) of the first aspect of the invention.

According to the present invention there is no particular limitation regarding the apparatus used for producing the organic diester. However, a reaction device at normal pressure and with stirring during reaction is preferably used for the synthesis step of monoepoxide ; for the synthesis reactions of diol, organic monoester and organic diester, a reaction apparatus equipped with a distillation device which may be under reduced pressure is preferably used.

The diolefins used as starting materials for the production of organic diester are diolefins which are generally available on the market (for example, product having a purity of 98%) ; for example, butadiene, pentadiene, hexadiene, heptadiene and the like may be used. More exactly, there are preferably few impurities and a small amount thereof in the diolefin used as starting material. Among them, 1, 3-butadiene is preferably used because it is easy to control synthesis of byproduct due to the reaction mechanism.

The peracid compounds used in the present invention are selected from the group consisting of hydrogen peroxide, performic acid, peracetic acid and perpropionic acid or among mixtures of at least two compounds chosen in this group. Among them, hydrogen peroxide and peracetic acid are preferably used and more preferably peracetic acid is used.

As carboxylic acids used in the present invention, formic acid, acetic acid, propionic acid, trifluoroacetic acid and butyric acid can be given as examples. Preferably at least one carboxylic acid selected from the group of formic acid, acetic acid and propionic acid is used. Preferably, acetic acid or propionic acid is used. More preferably, acetic acid is used.

The anhydrides used in the present invention are selected from the group of formic anhydride, acetic anhydride, propionic anhydride and mixture of at least two compounds of this group. Further, acetic anhydride and propionic anhydride are preferably used. More preferably, acetic anhydride is used.

In the monoepoxide synthesis reaction step which forms the first aspect of the preparing method of the invention, the starting diolefin and peracid compound are preferably introduced in a molar ratio of peracid compound / diolefin = 0.1 to 5, preferably from 0.5 to 3 and more preferably in the range of 0.7 to 2. When this ratio is less than 0.1, diolefin which has not reacted remains in a large amount and treatment for the unreacted gas becomes expensive. Further, when this ratio is more than 5, a large amount of peracid compound remains after reaction and the reaction is not stable. The solvent used in this reaction for dissolving diolefin may be, for example, ethyl formate, ethyl acetate, ethyl propionate, ethyl trifluoroacetate, ethyl butyrate. Preferably, ethyl acetate, ethyl propionate are used and more preferably ethyl acetate is used.

The reaction temperature is preferably in the range of 35°C to 70°C and more preferably in the range of 40°C to 65°C and most preferably in the range of 45°C to 60°C. When the reaction temperature is less than 35°C, the reaction is extremely delayed. On the other hand, when the reaction temperature is more than 70°C, the reaction temperature is then higher than the boiling point of the monoepoxide synthesized and thus there is a loss of this synthesized product.

The reaction pressure can be controlled via a pressure-control device and is preferably in the range of 0.5 to 10.0kgf/cm² G (1.5 x 10⁵ to 10.8 x 10⁵ Pa) (absolute pressure) as mentioned hereinafter), more preferably from 0.6 and less than 2.0 kgf/cm² G (1.6 x 10⁵ to 3.0 x10⁵ Pa) and more preferably from 0.7 to 1.5 kg f/cm² G (1.7 x 10⁵ to 2.5 x 10⁵ Pa). When the reaction pressure is less than 0.5 kg f/cm² G (1.5 x 10⁵ Pa) the reaction is delayed. On the other hand, when the reaction pressure is more than 10.0 kg f/cm² G (10.8 x 10⁵ Pa), a special apparatus able to resist at high pressure is then required and the installation for the reaction becomes expensive.

In the step (2), monoepoxide and water/carboxylic acid are reacted and a synthesis reaction of a diol with an organic monoester occurs. The molar ratio of carboxylic acid to be introduced with respect to the monoepoxide is preferably in the range of 0.1 to 20 (carboxylic acid / monoepoxide) and more preferably from 0.5 to 15 and most preferably in the range of 0.1 to 10. When this ratio is less than 0.1, unreacted monoepoxide remains in a large amount and treatment of unreacted gas becomes expensive. Further, when this ratio is more than 20, there is an excess of carboxylic acid and the reaction is delayed.

*It is to be noted that the carboxylic acid used in the process is preferably the same as the carboxylic acid produced from the percarboxylic acid which is used as a peroxide in the first step.* However, in this case, the theoretical amount of carboxylic acid synthesized in the first step (epoxidation step) is added to the amount of carboxylic acid to be introduced in the second step. This can also be suitably applied to the second and third object of the invention.

In the process, it is necessary to add water in order to increase the reactivity between monoepoxide and carboxylic acid. In this case, the molar ratio of water to be added with respect to monoepoxide is preferably water / monoepoxide = 0.1 to 10, more preferably from 0.3 to 7 and most preferably in the range of 0.5 to 5. When this ratio is less than 0.1, the reaction rate of the diol synthesis occasionally becomes extremely low. Further, when this ratio is more than 10, the effect consisting in greatly increasing the reaction rate due to the addition of water is lowered. Further, an increased energy for water treatment is then required. The molar ratio of water / carboxylic acid used in this process is preferably in the range of 0.1 to 10 and more preferably in the range of 0.2 to 7 and most preferably from 0.5 to 2.

The reaction temperature is preferably in the range of 50°C to 130°C and most preferably in the range of 70°C to 120°C and most preferably in the range of 80°C to 110°C. When the reaction temperature is lower than 50°C, the reaction rate of the reaction is greatly decreased. Further, when the reaction temperature is higher than 130°C, the reaction temperature at the beginning of the process is higher of more than 50° C than the boiling point of the monoepoxide and thus this causes losses of the starting monoepoxide.

The reaction pressure is preferably in the range of 0.5 to 2.0 kg f/cm² G (1.5 x 10⁵ to 3.0 x 10⁵ Pa), more preferably from 0.6 to 1.7 kg f/cm² G (1.6 x 10⁵ to 2.7 x 10⁵ Pa) and most preferably in the range of 0.7 to 1.5 kg f/cm² G (1.7 x 10⁵ to 2.5 x 10⁵ Pa). When the reaction pressure is less than 0.5 kg f/cm² G (1.5 x 10⁵ Pa), the reaction rate becomes low. Further, when the reaction pressure is higher than to 2.0 kg f/cm² G (3.0 x 10⁵ Pa), a special equipment which resists to high pressure reaction is required and thus the reaction equipment becomes expensive.

In this process, it is possible to remove by distillation after reaction solvents and distillates which have a boiling point lower than the boiling point of the organic monoester. The distillation apparatus needed for this distillation has preferably a theoretical plate-number of not less than 10 plates.

In the third step (3) which consists of allowing a monoester to react with an anhydride (also called an organic diester synthesis step) the molar ratio of the acid anhydride with respect to the organic monoester is preferably in the range of the acid anhydride / the organic monoester = 0.1 to 15, more preferably from 0.5 to 10 and most preferably from 1.0 to 5.0. When this ratio is lower than 0.1, there is a large amount of unreacted organic monoester and the yield of organic diester occasionally becomes extremely low. Further, when this ratio is higher than 15, there is an excess of the acid anhydride and the reaction is occasionally delayed. In this reaction, in order to reduce the loss of the acid anhydride, it is possible to recycle the unreacted acid anhydride to use it again.

The reaction temperature is from 100°C to 200°C, preferably from 120°C to 180°C and more preferably from 140°C to 170°C. When the reaction temperature is lower than 100°C, the reaction is extremely delayed. Further, when the reaction temperature is more than 200°C, bumping occurs in the distillation column which equipped the installation. Preferably, the temperature is set in order to obtain a reflux of the organic monoester and the acid anhydride.

The reaction pressure is preferably in the range of 0.5 to 2.0 kg f/cm² G (1.5 x 10⁵ to 3.0 x 10⁵ Pa), more preferably from 0.6 to 1.7 kg f/cm² G (1.6 x 10⁵ to 2.7 x 10⁵ Pa) and most preferably in the range of 0.7 to 1.5 kg f/cm² G (1.7 x 10⁵ to 2.5 x 10⁵ Pa). When the reaction pressure is less than 0.5 kg f/cm² G (1.5 x 10⁵ Pa), the reaction is occasionally delayed. Further, when the reaction pressure is higher than to 2.0 kg f/cm² G (3.0 x 10⁵ Pa), a special equipment which resists to high pressure reaction is required and thus the reaction equipment becomes expensive.

In this process, it is possible, to remove by distillation after reaction solvents and distillates which have a boiling point lower than the boiling point of the organic monoester by distillation. The distillation apparatus needed for this distillation has preferably a theoretical plate-number of not less than 10 plates. Moreover, in the reaction of each step of the process of the invention, it is possible to produce the organic diester without using any catalysts. Further, when 1,3-butadiene is used as a diolefin, acetic acid is used as a carboxylic acid and acetic anhydride is used as an acid anhydride, it is possible to obtain selectively 3, 4-diacetoxy-1-butene. Until now, there was no example of such unexpected results that can be obtained through the process of the present invention.

In the second aspect of the present invention, the starting materials used are the same as those used in the first aspect of the present invention. The reaction conditions of the first step (1) are the same as in the first aspect of the present invention. In the second step, a diol and an organic monoester are synthesized via reaction of a monoepoxide with water/a carboxylic acid. In this second step (2), in order to accelerate the synthesis of diol and also to increase the total number of moles of the diol and the organic monoester synthesized, it is necessary to add a number of moles of water greater than the number of mole of the carboxylic acid. In this case, the molar ratio of water added with respect to the monoepoxide is preferably in the range of water/monoepoxide = 0.1 to 50, more preferably in the range of 1 to 40 and most preferably in the range of 5 to 30.When this molar ratio is less than 0.1, the reaction rate of the diol synthesis becomes extremely low. Moreover, when this molar ratio is more than 50, a greater energy is required for water treatment in the following step.

The molar ratio of the carboxylic acid introduced with respect to the monoepoxide acid is preferably carboxylic / monoepoxide = 0.1 to 20, more preferably from 0.3 to 15 and most preferably from 0.5 to 10. When this molar ratio is less than 0.1, a lot of unreacted monoepoxide remains and cost of unreacted gas treatment become expensive. When this ratio is more than 20, the reaction is delayed because of an excess of the carboxylic acid. In this process, the molar ratio of water / carboxylic acid is more than 2.0 and less than 20 and preferably more than 2 and less than 10.

The reaction temperature is preferably in the range of 5°C to 130°C, more preferably in the range of 15°C to 110°C and most preferably in the range of 20°C to 100°C. When the reaction temperature is lower than 5°C, the reaction rate is extremely lowered. Further, when the reaction temperature is more than 130°C, the reaction temperature during the beginning of the reaction is higher in more than 50° C than the boiling point of monoepoxide and thus this involves loss of monoepoxide.

The reaction pressure is preferably in the range of 0.5 to 2.0 kg f/cm² G (1.5 x 10⁵ to 3.0 x 10⁵ Pa), more preferably from 0.6 to 1.7 kg f/cm² G (1.6 x 10⁵ to 2.7 x 10⁵ Pa) and most preferably in the range of 0.7 to 1.5 kg f/cm² G (1.7 x 10⁵ to 2.5 x 10⁵ Pa). When the reaction pressure is less than 0.5 kg f/cm² G (1.5 x 10⁵ Pa), the reaction rate becomes low. Further, when the reaction pressure is higher than 2.0 kg f/cm² G (3.0 x 10⁵ Pa), a special equipment which resists to high pressure reaction is required and thus the reaction equipment becomes expensive. In this process, it is possible to remove by distillation after reaction solvents and distillates having a boiling point lower than the boiling point of the organic monoester by distillation. The distillation apparatus needed for this distillation has preferably a theoretical plate-number of not less than 10 plates.

In the third step (3) wherein the diol and the organic monoester are allowed to react with an acid anhydride (this step corresponds to an organic diester synthesis reaction step), the molar ratios of diol and monoester with respect to the acid anhydride are both preferably In the range of the acid anhydride / diol and the acid anhydride / organic monoester of 0.1 to 15, preferably in the range of 0.5 to 10 and more preferably in the range of 1.0 to 5.0. When each of these molar ratios are less than 0.1, a lot of unreacted diol and organic monoester remain and the yield of organic diester becomes extremely low. Moreover, when each of these ratios is higher than 15, the reaction is delayed because of an excess of the acid anhydride.

In this reaction, in order to reduce the acid anhydride loss, it is possible to recycle unreacted acid anhydride and to use it again.

The reaction temperature is preferably in the range of 100°C to 200°C and more preferably in the range of 140°C to 170°C. When the reaction temperature is lower than 100°C, the reaction rate is extremely lowered. Further, when the reaction temperature is more than 200°C, bumping occurs in the distillation column which equipped the installation. Preferably, the temperature is set in order to obtain a reflux of diol and organic monoester and acid anhydride.

The reaction pressure can be controlled via a pressure-control device and is preferably in the range of 0.5 to 2.0kgf/cm² G (1.5 x 10⁵ to 3.0 x 10⁵ Pa), more preferably from 0.6 to 1.7 kgf/cm² G (1.6 x 10⁵ to 2.7 x 10⁵ Pa) and most preferably from 0.7 to 1.5 kg f/cm² G (1.7 x 10⁵ to 2.5 x 10⁵ Pa). When the reaction pressure is less than 0.5 kg f/cm² G (1.5 x 10⁵ Pa) the reaction is delayed. Moreover, when the reaction pressure is more than 2.0 kg f/cm² G (10.8 x 10⁵ Pa), a special apparatus able to resist at high pressure is then required and the installation for the reaction becomes expensive. In this process, it is possible to remove by distillation after reaction solvent and distillates having boiling points lower than the boiling point of the organic monoester by distillation. The distillation apparatus needed for this distillation has preferably not less than 10 theoretical plates. For every reaction of the process of the present invention, it is possible to produce an organic diester without using any catalysts.

Moreover, when 1,3-butadiene is used as a diolefin, acetic acid is used as a carboxylic acid and acetic anhydride is used as an acetic anhydride, it is possible to obtain selectively 3, 4-diacetoxy-1-butene. Until now there was no example of such unexpected results that can be obtained through the process of the present invention. Further, according to the process of the invention, it is possible to largely reduce the amount of waste products because of the high selectivity of the process of the invention. The process of the invention enables a production which is environmentally-friendly.

In the third aspect of the invention, the starting materials are the same as those used in the first step (1) of the first aspect of the invention. The reaction conditions of the first step (1) which consists of a monopeoxide production step are the same as the reaction conditions for the first step (1) in the first aspect of the invention. In the third aspect of the invention, the reaction mixture which contains the monoepoxide synthesized is allowed to react with water to synthesize a diol. At this time, in the case of, for example, using the peracid of the first step (1), there is produced an organic monoester due to production of acetic acid. The molar ratio of water with respect to the monopoxide to be introduced is preferably water / monoepoxide from 1 to 40, more preferably from 5 to 30 and most preferably from 10 to 20. When this molar ratio is less than 1, the reaction rate of the diol synthesis becomes low. Further, when this ratio is more than 40, a water excessively remaining after reaction has to be removed, a great energy is required for the process and thus the production cost increases.

The reaction temperature is preferably in the range of 50°C to 130°C, more preferably in the range of 70° C to 120° C and most preferably in the range of 80°C to 110°C. When the reaction temperature is lower than 50°C, the reaction rate is extremely lowered. Further, when the reaction temperature is more than 130°C, the reaction temperature during the beginning of the reaction is higher of more than 50° C than the boiling point of monoepoxide and thus this involves loss in monoepoxide which is a starting material.

The reaction pressure can be controlled via a pressure-control device. The ranges of the reaction pressure are the same as the step (2) in the first and second aspect of the present invention. In this reaction, it is possible to remove solvent and distillates having boiling points lower than the boiling point of the organic monoester by distillation after reaction. The distillation apparatus needed for this distillation has preferably not less than 10 theoretical plates.

In the third step (corresponds to the diol synthesis step) where diol and an acid anhydride are reacted, the molar ratio of the acid anhydride with respect to the diol to be introduced is preferably: acid anhydride / diol from 0.1 to 15, more preferably from 0.5 to 10 and most preferably from 1.0 to 5.0. When this molar ratio is less than 0.1, there is a lot of unreacted diol and the yield becomes extremely low. Further, when this ratio is more than 15, the reaction rate is delayed because of an excess amount of the acid anhydride.

In this reaction, it is possible to use the recycled unreacted anhydride compound in order to reduce as much as possible the small lasses of acid anhydride.

The conditions regarding the reaction temperature and pressure of the reaction are the same as these in the third step (3) of the first and second aspect of the invention.

In this reaction, it is possible to remove fractions having a boiling point lower than the boiling point of the organic diester by distillation after reaction. For this purpose, the distillation apparatus has preferably a theoretical plates number of not less than 10. Moreover, in each reaction of the process of the present invention, it is possible to produce an organic diester without using catalysts.

Further, when 1,3-butadiene is used as a diolefin, acetic acid is used as a carboxylic acid and acetic anhydride is used as an acetic anhydride, it is possible to obtain selectively 3, 4-diacetoxy-1-butene. Until now there was no example of such unexpected results that can be obtained through the process of the present invention.

The fourth aspect of the invention relates to a method of producing an organic diester producing method comprising a first step (1) which is a monopeoxide synthesis step, the monoepoxide being obtained from allowing a diolefin to react with a peracid compound, a second step (2) in which the monoepoxide synthesized in the first step is allowed to react with water or water and carboxylic acid and a third step (3) in which the synthesized product is allowed to an anhydride compound. According to the fourth aspect of the invention, this method is characterized in that water and the carboxylic acid are removed from the synthesized product of the second step (2) and recycled to be used in the second step (2). The purpose of the present invention is to reduce a load involved by water treatment required after reaction and particularly to reduce the amount of acetic acid used in the above-mentioned method of producing an organic diester wherein peracetic acid is used as a peracid compound and acetic acid as a carboxylic acid.

The starting materials are the same as those used in the first step (1) of the first aspect of the invention. Further, the reaction conditions of the first step (1) are the same as those in the first step (1) of the first aspect of the invention. In the second step (2), it is possible to use only water to be reacted with the monopeoxide. When only water is used, the reaction conditions can be selected on the basis of the conditions of the third aspect of the invention. When water and carboxylic acid are used, the reaction conditions may be selected on the basis on the first or the second aspect of the present invention.

Organic monoester, diol, unreacted monoepoxide, solvent, water, unreacted peracid, carboxylic acid and other by-products are contained in the synthesized product coming from the reaction between water or water and carboxylic acid and monoepoxide.

In this step, it is possible to remove a solvent, water, a carboxylic acid and other components having a boiling point lower than the boiling points of organic monoester and diol by distillation. Water containing carboxylic acid is recycled and the recycled water and carboxylic acid are used again in the second step (2). The method for the preparation is preferably used in the case that the monoepoxide is allowed to react with water and carboxylic acid in the molar ratio of (water/carboxylic acid) of 2 to 20. Further, as suitable examples of starting materials, butadiene can be used as a diolefin, peracetic acid can be used as a peracid, acetic acid can be used as carboxylic acid and acetic anhydride can be used as an anhydride compound. In the above-mentioned first, second and third aspects of the invention, energy is required to the treatment of waste water. However, in the second step (2), since water and carboxylic acid are recycled and used again in the second step (2), the load of waste water is decreased and use amount of acetic acid is also decreased.

### EXAMPLES

Hereinafter, the present invention will be explained in more details on the basis of the following examples and comparative examples. However, the present invention is not limited to these examples. Hereinafter, the symbol % refers to weight percent. The following evaluations and measuring methods were employed.
1. Regarding the identification of respective compounds and the determination of the amounts, an analysis using gas chromatography was undergone. Further, regarding by-products, a qualitative analysis using 1H-NMR, 13C-NMR and a gas chromatography mass analysis device was conducted.
2. selectivity: *method based on the internal standard method of gas chromatography and calculation according to an area percentage method*

### EXAMPLE 1

693 g of butadiene, 3247g of a solution of ethyl acetate containing 30% of peracetic acid and 1617g of ethyl acetate used as a solvent were introduced in a reaction apparatus and mixed together. The reaction temperature was set in a range from 45°C to 60°C, the reaction pressure was set at 1kg/cm² G (2.0 x 10⁵ Pa) and the reaction was continued for 5 hours to produce a butadienemonoepoxide. Then, 2650g of acetic acid and 960g of water were added and the reaction temperature and the reaction pressure were set in a range from 90°C to 100°C and 1kg/cm² G (2.0 x 10⁵ Pa), respectively, to carry out the butadienemonoacetate synthesis over 2 hours. During the reaction, 3, 4-dihydroxy-1-butene was also synthesized by the reaction between water and butadienemonoacetate. After the reaction of more esterification, solvent components having a boiling point lower than the boiling points of butadienemonoacetate, and 3, 4-dihydroxy-1-butene were removed through distillation. The above-described operations (from the beginning to distillation) were conducted twice and then 2500g of acetic anhydride was added to the total remaining fraction. The resulting mixture was set while refluxing at 160°C to synthesize butadienediacetate. As a result, after 3 hours from the beginning of the diesterification reaction, the yield of 3, 4-diacetoxy-1-butene was of 55% based on butadiene. During the reaction, butadienemonoacetate which was synthesized during the monoesterification reaction and 3, 4-dihydroxy-1-butene were converted into 3, 4-diacetoxy-1-butene with a selectivity of not less than 95%.

### EXAMPLE 2

The same operations as described in Example 1 were carried out, except that 5300g of acetic acid and 960g of water were added. As a result, after 3 hours from the beginning of the diesterification reaction, the yield of 3, 4-diacetoxy-1-butene was of 53% based on butadiene. During the reaction, butadienemonoacetate which was synthesized during the monoesterification reaction and 3, 4-dihydroxy-1-butene were converted into 3, 4-diacetoxy-1-butene with a selectivity of not less than 95%.

### EXAMPLE 3

The same operations as described in Example 1 were carried out except that 2650g of acetic acid and 1920g of water were added. As a result, after 3 hours from the beginning of the diesterification reaction, the yield of 3, 4-diacetoxy-1-butene was of 51% based on butadiene. During the reaction, butadienemonoacetate which was synthesized during the monoesterification reaction and 3, 4-dihydroxy-1-butene were converted into 3, 4-diacetoxy-1-butene with a selectivity of not less than 95%.

### COMPARATIVE EXAMPLE 1

693g of butadiene, 3247g of a solution of ethyl acetate containing 30% of peracetic acid, 1617g of ethyl acetate, 2650g of acetic acid, 960g of water and 2500g of acetic anhydride were introduced in a reaction apparatus and mixed each other. Butadienediacetate synthesis reaction was undertaken at a reaction temperature in the range of 90°C to 100°C and with a reaction pressure of 1kg f/cm² G (2.0 x 10⁵ Pa).

As a result, after 3 hours from the beginning of the reaction, the yield of 3, 4-diacetoxy-1-butene was of 19% based on butadiene.

### COMPARATIVE EXAMPLE 2

20g of an ion-exchange resin was added to the starting material of Comparative Example 1 and the synthesis reaction butadienediacetate was undertaken at a reaction temperature of 90°C to 100°C and under a reaction pressure of 1kg f/cm² G (2.0 x 10⁵ Pa).

As a result, after 3 hours from the beginning of the reaction, the yield of 3, 4-diacetoxy-1-butene was of 21% based on butadiene.

### EXAMPLE 4

598 g of butadiene, 2806g of a solution of ethyl acetate containing 30% of peracetic acid and 1617g of ethyl acetate used as a solvent were introduced in a reaction apparatus and mixed each other. The reaction temperature was set in the range from 45°C to 60°C, the reaction pressure was set at 1 kg/cm² G (2.0 x 10⁵ Pa) and the reaction was continued for 5 hours to produce a butadienemonoepoxide. Then, 1076g of acetic acid and 1794g of water were added and the reaction temperature and the reaction pressure were set in a range from 50°C to 90°C and 1kg/cm² G (2.0 x 10⁵ Pa), respectively to carry out synthesis of 3, 4-dihydroxy-1-butene, 3-hydroxy-4-acetoxy-1-butene and 3-acetoxy-4-hydroxy-1-butene synthesis within 2 hours. After this reaction, the solvent and the components having a boiling point lower than the boiling points of the above-mentioned synthesized products were removed through distillation. The above-described operations (from the beginning to distillation) were repeated twice and then 3500 g of acetic anhydride was added to the total remaining fraction. The resulting mixture was refluxed at 160°C and butadienediacetate was obtained. As a result, after 3 hours from the beginning of the diesterification reaction, the yield of 3, 4-diacetoxy-1-butene was of 55% based on butadiene. During the reaction, 3, 4-dihydroxy-1-butene, 3-hydroxy-4-acetoxy-1-butene and 3-acetoxy-4-hydroxy-1-butene were converted into 3, 4-diacetoxy-1-butene with a selectivity of not less than 95%.

### EXAMPLE 5

The same reaction was carried out as explained in Example 4, except that during the synthesis of 3, 4-dihydroxy-1-butene, 3-hydroxy-4-acetoxy-1-butene and 3-acetoxy-4-hydroxy-1-butene, 1076g of acetic acid and 3588g of water were added. As a result, after 3 hours from the beginning of the diesterification reaction, the yield of 3, 4-diacetoxy-1-butene was of 52% based on butadiene. During the reaction, 3, 4-dihydroxy-1-butene, 3-hydroxy-4-acetoxy-1-butene and 3-acetoxy-4-hydroxy-1-butene were converted into 3, 4-diacetoxy-1-butene with a selectivity of not less than 95%.

### EXAMPLE 6

The same reaction was carried out as explained in Example 4, except that during the synthesis of 3, 4-dihydroxy-1-butene, 3-hydroxy-4-acetoxy-1-butene and 3-acetoxy-4-hydroxy-1-butene, 2152g of acetic acid and 1794g of water were added. As a result, after 3 hours from the beginning of the diesterification reaction, the yield of 3, 4-diacetoxy-1-butene was of 49% based on butadiene. During the reaction, 3, 4-dihydroxy-1-butene, 3-hydroxy-4-acetoxy-1-butene and 3-acetoxy-4-hydroxy-1-butene were converted into 3, 4-diacetoxy-1-butene with a selectivity of not less than 95%.

### COMPARATIVE EXAMPLE 3

598g of butadiene, 2806g of a solution of ethyl acetate containing 30% of peracetic acid, 1617g of ethyl acetate, 1076g of acetic acid, 1794g of water and 3500g of acetic anhydride were introduced in a reaction apparatus and mixed each other. Butadienediacetate synthesis reaction was undertaken at a reaction temperature in the range of 50°C to 90°C and with a reaction pressure of 1kg f/cm² G (2.0 x 10⁵ Pa). As a result, after 3 hours from the reaction, the yield of 3, 4-diacetoxy-1-butene was of 19% based on butadiene.

### COMPARATIVE EXAMPLE 4

20g of an ion-exchange resin was added to the starting material of Comparative Example 3 and the synthesis reaction butadienediacetate was undertaken at a reaction temperature of 50°C to 90°C and under a reaction pressure of 1kg f/cm² G (2.0 x 10⁵ Pa). As a result, after 3 hours from the beginning of the reaction, the yield of 3, 4-diacetoxy-1-butene was of 21% based on butadiene.

### EXAMPLE 7

693 g of butadiene, 3247g of a solution of ethyl acetate containing 30% of peracetic acid and 1617g of ethyl acetate used as a solvent were introduced in a reaction apparatus and mixed each other. The reaction temperature was set in the range from 45°C to 60°C, the reaction pressure was set at 1kg/cm² G (2.0 x 10⁵ Pa) and the reaction was continued for 5 hours to produce a butadienemonoepoxide. Then, 3113g of water were added and the reaction temperature and the reaction pressure were set in a range from 90°C to 100°C and 1kg/cm² G (2.0 x 10⁵ Pa), respectively, to carry out a synthesis reaction of 3, 4-dihydroxy-1-butene. At this time, butadienemonoacetate is also synthesized through the reaction between butadienemonoepoxyde and remaining acetic acid. After the second step, solvent and components having a boiling point lower than the boiling points of butadienemonoacetate and 3, 4-dihydroxy-1-butene were removed through distillation. After adding two batch products obtained through the same operations as explained above, 2500g of acetic anhydride was added to these two batch products and synthesis of butadienediacetate was carried out under reflux at 160°C. As a result, after 3 hours from the beginning of the third step, the yield of 3, 4-diacetoxy-1-butene was of 55% based on butadiene. At this moment, butadienemonoacetate and 3, 4-dihydroxy-1-butene which were synthesized during the second-step reaction were converted into 3, 4-diacetoxy-1-butene with a selectivity of not less than 95%.

### EXAMPLE 8

The same operations as explained in Example 7 were carried out except that during the second-step reaction 2075g of water was added. As a result, after 3 hours from the beginning of the third step reaction, the yield of 3, 4-diacetoxy-1-butene was of 53% based on butadiene. At this time, 3, 4-dihydroxy-1-butene and butadienemonoacetate which were synthesized during the second-step reaction were converted into 3, 4-diacetoxy-1-butene with a selectivity of not less than 95%.

### EXAMPLE 9

The same operations as explained in Example 7 were carried out except that during the second-step reaction 4150g of water was added. As a result, after 3 hours from the beginning of the third step reaction, the yield of 3, 4-diacetoxy-1-butene was of 51% based on butadiene. At this time, 3, 4-dihydroxy-1-butene and butadienemonoacetate which were synthesized during the second-step reaction were converted into 3, 4-diacetoxy-1-butene with a selectivity of not less than 95%.

### COMPARATIVE EXAMPLE 5

693g of butadiene, 3247g of a solution of ethyl acetate containing 30% of peracetic acid, 1617g of ethyl acetate, 2650g of acetic acid, 960g of water and 2500g of acetic anhydride were introduced in a reaction apparatus and mixed each other. A synthesis reaction of butadienediacetate was undertaken at a reaction temperature in the range of 90°C to 100°C and with a reaction pressure of 1kg f/cm² G (2.0 x 10⁵ Pa). As a result, after 3 hours from the reaction, the yield of 3, 4-diacetoxy-1-butene was of 19% based on butadiene.

### COMPARATIVE EXAMPLE 6

20g of an ion-exchange resin was added to the starting material of Comparative Example 5 and a synthesis reaction of butadienediacetate was undertaken at a reaction temperature of 90°C to 100°C and under a reaction pressure of 1kg f/cm² G (2.0 x 10⁵ Pa). As a result, at the period of 3 hours after the beginning of the reaction, the yield of 3, 4-diacetoxy-1-butene was of 21% based on butadiene.

### INDUSTRIAL POSSIBLE ADVANTAGES

According to the first, second and third aspects of the present invention, it is possible to carry out a reaction with high reactivity and selectivity and in the same time without using any catalyst Thus, a production without any catalyst treatment is possible. Further, it was possible to obtain a pioneering result that until now there was no example of obtaining selectively 3, 4-diacetoxy-1-butene by using 1, 3-butadiene as a diolefin, acetic acid as a carboxylic acid and acetic anhydride as an acid anhydride. Moreover, according to the fourth aspect of the present invention, in the second step (2) thereof, water and carboxylic acid are recycled and used again in the second step (2). Thus, it is possible, for example when using the above-mentioned starting material to reduce the load of dumping water and the amount of acetic acid used.

## Claims

1. A method of preparing an organic diester characterized in that said method comprises a first step (1) where a monoepoxide is synthesized by allowing to react a diolefin with a peracid compound, a second step (2) where said monoepoxide is reacted with water and carboxylic acid and a third step (3) where the synthesized products are reacted with an acid anhydride.

2. A method of preparing an organic diester of claim 1, characterized in that in the second step (2), the molar ratio of water and carboxylic acid reacted with said monoepoxide is not more than (water/carboxylic acid) 0.1 to 2.

3. A method of preparing an organic diester of claim 1 or 2, characterized in that said diolefin is butadiene.

4. A method of preparing an organic diester of claim 3, characterized in that said peracid compound is at least one compound selected from the group consisting of hydrogen peroxide, performic acid, peracetic acid and perpropionic acid.

5. A method of preparing an organic diester of claim 4, characterized in that said carboxylic acid is at least one selected from the group consisting of formic acid, acetic acid and propionic acid.

6. A method of preparing an organic diester of claim 5, characterized in that said anhydride is at least one selected from the group consisting of formic anhydride, acetic anhydride and propionic anhydride.

7. A method of preparing an organic diester of claim 1 or 2, characterized in that said diolefin is 1, 3-butadiene, said peracid compound is peracetic acid, said carboxylic add is acetic acid and said acid anhydride is acetic anhydride.

8. A method of preparing an organic diester characterized in that said method comprises a first step (1) where a monoepoxide is synthesized by allowing to react a diolefin with a peracid compound, a second step (2) where said monoepoxide is reacted with water and carboxylic acid in a molar ratio of more than 2 and not more than 20 (water/carboxylic acid) and a third step (3) where the synthesized products are allowed to react with an acid anhydride.

9. A method of preparing an organic diester of claim 8, characterized in that said diolefin is butadiene.

10. A method of preparing an organic diester of claim 9, characterized in that said peracid compound is at least one compound selected from the group consisting of hydrogen peroxide, formic acid, peracetic acid and perpropionic acid.

11. A method of preparing an organic diester of claim 9, characterized in that said carboxylic acid is at least one selected from the group consisting of formic acid, acetic acid and propionic acid.

12. A method of preparing an organic diester of claim 11, characterized in that said acid anhydride is at least one selected from the group consisting of formic anhydride of acetic anhydride and propionic anhydride.

13. A method of preparing an organic diester of claim 12, characterized in that said diolefin is 1, 3-butadiene, said peracid compound is peracetic acid, said carboxylic acid is acetic add and said acid anhydride is acetic anhydride, said organic diester is 3, 4-diacetoxy-1-butene.

14. A method of preparing an organic diester characterized in that said method comprises a first step (1) where a monoepoxide is synthesized by allowing to react a diolefin with a peracid compound, a second step (2) where said monoepoxide is reacted with water and a third step (3) where the synthesized products are reacted with an acid anhydride.

15. A method of preparing an organic diester of claim 14, characterized in that said diolefin is butadiene.

16. A method of preparing an organic diester of claim 15, characterized in that said peracid compound is at least one selected from the group consisting of hydrogen peroxide, formic acid, peracetic acid and perpropionic acid.

17. A method of preparing an organic diester of claim 16, characterized in that said anhydride is at least one selected from the group consisting of formic anhydride of acetic anhydride and propionic anhydride.

18. A method of preparing an organic diester of claim 14, characterized in that said diolefin is 1, 3-butadiene, said peracid compound is peracetic acid, said carboxylic acid is acetic acid and said acid anhydride is acetic anhydride, said organic diester is 3, 4-diacetoxy-1-butene.

19. A method of preparing an organic diester comprising a first step (1) where a monoepoxide is synthesized by allowing to react a diolefin with a peracid compound, a second step (2) where said monoepoxide is reacted with water or water and carboxylic acid and a third step (3) where the synthesized products are reacted with an acid anhydride, said method being characterized in that in the second step (2) water and said carboxylic acid are removed from the synthesized products of said second step and recycled to be used again in the reaction of said second step (2).

20. A method of preparing an organic diester of claim 19, characterized in that in the second step (2), the molar ratio of water and carboxylic acid (water/carboxylic acid) is more than 2 and not more than 20.

21. A method of preparing an organic diester of claim 19, characterized in that said diolefin is 1, 3-butadiene, said peracid compound is peracetic acid, said carboxylic acid is acetic acid and said acid anhydride is acetic anhydride, said organic diester is 3, 4-diacetoxy-1-butene.
